# EUROPÄISCHE PATENTSCHRIFT

(11) **EP 0 503 252 B1**
(45) Veröffentlichungstag und Bekanntmachung des Hinweises auf die Patenterteilung: **04.04.2001**
(21) Anmeldenummer: 92101272.0
(22) Anmeldetag: 27.01.1992
(51) Int. Cl.: C12N 15/81, C12N 15/51, C12N 15/36, G01N 33/569, A61K 39/29, C12P 21/08

(54) **Rekombinante Proteine mit der Immunreaktivität des Hepatitis B Virus e Antigens (HBeAg), Verfahren zu ihrer Herstellung und ihre Verwendung in Immunoassays und Impfstoffen**
Recombinant proteins having the immunoreactivity of Hepatitis B virus e antigens (HBeAg), method for their production and their application in immunoassays and as vaccines
Protéines recombinantes ayant l'activité immunologique de l'antigène e du virus de l'hépatite B (HBeAg), procédé de leur production ainsi que leur utilisation dans des assays immunologiques et comme vaccins

(30) Priorität: 09.03.1991 DE 4107612
(43) Veröffentlichungstag der Anmeldung: 16.09.1992
(73) Patentinhaber: Dade Behring Marburg GmbH, 35001 Marburg (DE)
(72) Erfinder: Noah, Michael, W-3550 Marburg (DE); Bröker, Michael, W-3550 Marburg (DE)

(56) Entgegenhaltungen:
- EP-A- 0 374 869
- WO-A-90/08960
- WO-A-92/11368
- FR-A- 2 635 115
- JOURNAL OF VIROLOGY Bd. 59, Nr. 1, 1986, Seiten 176 - 180 MIYANOHARA ET AL. 'Expression of hepatitis B virus core antigen gene in Saccharomyces cerevisiae'
- JOURNAL OF VIROLOGY Bd. 61, Nr. 3, M{rz 1987, Seiten 683 - 692 MCLACHLAN A. ET AL. 'Expression of hepatitis B virus surface and core antigens'
- NUCLEIC ACIDS RESEARCH. Bd. 15, Nr. 24, 23. Dezember 1987, ARLINGTON, VIRGINIA US Seiten 10117 - 10132 JUNKER M. ET AL. 'Expression and replication of the hepatitis B virus genome'

## Beschreibung

Die Erfindung betrifft rekombinante Proteine mit der Immunreaktivität des Hepatitis B e Antigens (HBeAg), Verfahren zu ihrer Herstellung in Hefen und ihre Verwendung in Immunoassays und in Impfstoffen.

Immunoassays spielen heute bei der Diagnose von Hepatitis B Virus Infektionen eine wichtige Rolle. So wird die akute Phase einer Infektion durch den immunologischen Nachweis von Hepatitis B Virus Oberflächen-Antigen (HBsAg) charakterisiert. Die Bestimmung weiterer HBV-Parameter erlaubt die Absicherung der Diagnose bzw. die Differentialdiagnose. So geht man davon aus, daß HBsAg- und HBeAg-positive Proben akut infektiös sind, wohingegen das Auftreten von Antikörpern gegen das HBeAg (Anti-HBeAg-Antikörper) den Beginn der Rekonvaleszenzphase des Patienten markiert.

Zur Bestimmung des HBeAg bzw. von Anti-HBeAg-Antikörpern haben sich weltweit Radio- und Enzymimmunoassays durchgesetzt und andere weniger sensitive Techniken wie Agglutinationsmethoden weitgehend verdrängt. Diese Assays arbeiten nach dem "Sandwich-Prinzip". Eine feste Phase, z.B. die Näpfchen einer Mikrotitrationsplatte oder Kugeln, die mit menschlichen oder Maus-Anti-HBeAg-Antikörpern beschichtet sind, wird mit der Patientenprobe inkubiert. Ist HBeAg in der Probe enthalten, bindet es an die Festphasenantikörper. Nicht gebundene Bestandteile werden in einem Waschvorgang entfernt. Das an die Festphase gebundene HBeAg wird während einer zweiten Inkubation mittels eines mit einem Enzym oder Radioisotop gekoppelten Anti-HBeAg-Antikörpers markiert. Nach einem weiteren Waschvorgang wird durch die Umsetzung eines Substrats bzw. durch die Messung der Radioaktivität dieser ternäre Komplex nachgewiesen.

Für den Nachweis der Anti-HBeAg-Antikörper lassen sich die gleichen Reagenzien und das gleiche Testschema verwenden, wenn man zur Probe noch eine definierte Menge HBeAg (das sog. Neutralisationsreagenz) gibt. Sollten in dem Untersuchungsmaterial, in der Regel eine Serumprobe, keine Anti-HBeAg-Antikörper vorhanden sein, wird somit ein bestimmtes Signal erzeugt. Sind aber Anti-HBeAg-Antikörper in der Probe vorhanden, so binden diese an das HBeAg des Neutralisationsreagenz und verhindern dessen Bindung an die Festphase und somit auch die Bildung des Signals. Diese Art des Testaufbaus verbindet somit Aspekte des Sandwich- und des kompetitiven Testprinzips.

Testsysteme für die Bestimmung des HBeAg und der Anti-HBeAg-Antikörper nach den oben beschriebenen Prinzipien sind von mehreren Herstellern erhältlich und auch in der Literatur mehrfach beschrieben (z.B. Abbott HBe(rDNA), Wiesbaden; Behring ^{R}Enzygnost-HBe, Marburg; Sorin Biomedica EBK EIA, Düsseldorf).

Als Kontrolle für die korrekte Durchführung des Tests verwenden alle Testsysteme zur Bestimmung des HBeAg eine sogenannte positive Kontrolle, die eine bestimmte Menge HBeAg enthält und somit zu einem definierten Signal führen muß, wenn der Test korrekt durchgeführt wurde. Wird dieses Signal nicht erreicht, darf der Testlauf wegen offensichtlicher Durchführungsfehler nicht gewertet werden und muß wiederholt werden. Bei der Bestimmung der Anti-HBeAg-Antikörper ist das HBeAg im Neutralisationsreagenz sogar, wie oben erläutert, prinzipiell für die Testdurchführung notwendig.

Die bis dato etablierten Testsysteme verwenden zum Teil HBeAg, das aus dem Blut HBV-infizierter Menschen gewonnen werden mußte, da keine Zellkultursysteme zur Viruszucht etabliert werden konnten. Als nachteilig an diesem Material erweist sich die Schwierigkeit, größere Mengen hochtitrigen HBeAg-positiven Serums von den Infizierten zu erhalten.

Außerdem ist das HBeAg-positive Serum durch seine Infektionsgefährdung nur unter aufwendigen und teuren Sicherheitsvorkehrungen zu handhaben.

Als einziger Schutz vor einer Hepatitis B Infektion gilt heutzutage neben allgemeinen hygienischen Maßnahmen die Impfung.

In den zur Zeit kommerziell erhältlichen Impfstoffen wird als Immunogen lediglich HBsAg verwendet, doch gibt es in der Literatur seit einigen Jahren Hinweise, daß ein Impfschutz durch die Verwendung von HBcAg- und/oder HBeAg-Komponenten einzeln, als Mischung oder als Fusion mit einem weiteren Immunogen erzielt bzw. verbessert werden könnte. Für Immunisierungszwecke wäre es deshalb ebenfalls wichtig, ein HBeAg ohne Infektionspotential durch gentechnologische Methoden zu erzeugen, das außerdem gegenüber den bisher bekannten Materialien Vorteile durch optimale Immunreaktivität ohne weitere Denaturierungsmaßnahmen aufweisen müßte sowie einfach und preiswert in genügenden Mengen herstellbar sein sollte.

1979 und 1980 erschienen erste Arbeiten, die zeigten, daß HBcAg humanen Ursprungs durch Denaturierung in z.B. SDS einen großen Teil der HBc-Immunreaktivität verliert und dafür HBe-Immunreaktivität gewinnt (z.B. Takahashi et al., J. Immunol. 122 (1979), 275 - 279)
Diese Methode auf HBcAg humanen Ursprungs angewendet ergibt aber keinerlei Vorteile gegenüber dem HBeAg humanen Ursprungs, da die Probleme der Beschaffung und Infektiosität bestehen bleiben.

Nachdem es gelang, HBcAg mit gentechnologischen Methoden in E.coli zu exprimieren, wurde die Denaturierungstechnik auch mit dem rHBcAg durchgeführt. Nachteilig ist auch hier, daß eine gewisse HBcAg -Immunreaktivität immer noch in diesen Präparationen verbleibt.

In der EP-A 075 395 wurde dann ein verkürztes rekombinantes HBcAg (bis Aminosäure 144) aus E.coli beschrieben, das neben der HBcAg -Immunreaktivität auch HBeAg-Immunreaktivität aufwies. Die verbleibende HBcAg-Immunreaktivität mußte aber wieder durch Denaturierungsmaßnahmen beseitigt werden.

Nachdem Takahashi et al. (a.a.O.) zeigen konnten, daß das HBeAg in der C-terminalen Aminosäuresequenz dem HBcAg bis auf die fehlenden Aminosäuren ab Position 150 entspricht, wurde das verkürzte HBcAg auch als Fusionsprotein in E.coli exprimiert. Auch bei diesem Material mußte die verbleibende HBcAg-Immunreaktivität durch Denaturierung beseitigt werden (Mimms et al., Vir. Hepatitis and Liver Disease (1988), 248-251).

Zum Beginn der achtziger Jahre ging man also davon aus, daß HBeAg ein Denaturierungsprodukt und/oder ein Abbauprodukt des HBcAg darstellte. Es hat sich jedoch herausgestellt, daß vor dem Translationsstartsignal des HBcAg eine DNA-Sequenz mit einem offenen Leserahmen von 29 Aminosäuren liegt (pre C-Sequenz, Aminosäuren -29 bis -1).

Nach dem jetzigen Kenntnisstand ist davon auszugehen, daß in dem Bereich des HBcAg-Gens (pre c plus C-Sequenz) zwei verschiedene mRNA's abgelesen werden oder eine mRNA-Species codiert für zwei verschiedene Translationsprodukte. Die HBcAg-spezifische mRNA enthält einen offenen Leserahmen mit den Codons +1 bis +183 und führt durch die Translation zum HBcAg, das die Aminosäuren +1 bis +183 umfaßt.

Die HBeAg-spezifische mRNA hingegen enthält einen offenen Leserahmen mit den Codons -29 bis +183. Durch die Translation wird ein Vorläufermolekül gebildet, das die Aminosäuren -29 bis +183 enthält. Die ersten 19 Aminosäuren dieses Vorläuferproteins fungieren als Signalsequenz, die zur Sekretion des Vorläuferproteins ins endoplasmatische Retikulum (ER) führen. Im Verlauf der weiteren Prozessierung werden auch die C-terminalen Aminosäuren ab Position 150 proteolytisch abgespalten, so daß schließlich HBeAg in die Blutbahn sezerniert wird.

Trotz weitgehend identischer Aminosäuresequenz haben HBcAg und HBeAg vollkommen verschiedene immunologische, strukturelle und funktionelle Eigenschaften. Da auch die Transkription an verschiedenen Startpunkten beginnt und zu verschiedenen mRNA's führt, muß man davon ausgehen, daß HBcAg und HBeAg letztlich von unterschiedlichen Genen, wenn auch mit einer gewissen Überlappung, codiert werden.

Als Beispiel der Wichtigkeit der Signalsequenz für die Bildung des HBeAg sei hier die Arbeit von Kim et al. (The 1990 International Symposium on Viral Hepatitis and Liver Disease, Houston, Texas, April 4 - 8, 1990, poster abstract No. 62) erwähnt. Sie zeigt, daß man im Cytosol von Hefen nur HBcAg-reaktives Material erhält, wenn man HBeAg ohne die Signalsequenz direkt in Hefen exprimieren will und beschreibt, daß man das HBeAg an die alpha--Faktor-Signalsequenz fusionieren muß, wenn man Material erhalten will, das hauptsächlich HBeAg-Reaktivität aufweisen soll.

Die Sekretion mittels Signalsequenz des alpha-Faktors hat die Nachteile, daß diese Signalsequenz im Wirtsorganismus nicht gespalten wird oder falsch gespalten wird oder zum Abbau des sekretierten Protein führen kann, wobei diese Ereignisse in der Regel nebeneinander auftreten. Die Sezernierung von humanem Albumin erfolgt z.B. durch die Signalsequenz des alpha-Faktors schlechter als mit der proteineigenen Signalsequenz des Albumin oder mit einer Hybridsignalsequenz zwischen dem K. lactis Killertoxin and dem alpha-Faktor (Sleep et al., Bio/Technology (1990), 8, 42-46).

Demgegenüber wurde gefunden, daß die Expression von HBeAg ohne HBc-Reaktivität schon ohne Signal-Sequenz erfolgt, also zu HBeAg ohne HBc-Reaktivität und ohne Fremdproteinanteil führt. Weiter wurde gefunden, daß eine Sekretion bzw. Expression mittels der HBeAg-eigenen Signal-sequenz bewirkt wird, was möglicherweise ein HBeAg ergibt, das zusätzliche Bereiche bis zur Position -29 umfassen und damit zusätzliche prae-C-Epitope enthalten kann.

Erfindungsgemäß wird angestrebt, den vermuteten Prozeß, wie er in der infizierten Leberzelle stattfindet, nachzuahmen, um ein rekombinantes Protein mit größtmöglicher Ähnlichkeit bzw. Identität zum HBeAg des Humansystems - und damit optimaler Immunreaktivität - zu erhalten.

Erfindungsgemäß wird deshalb als Expressionssystem das eukaryontische Hefe-System verwendet, da es im Gegensatz zum E.coli-System über komplexere posttranslatorische Modifikationsmechanismen verfügt und somit erheblich ähnlicher zum eukaryontischen Humansystem ist. Andererseits ist die Expression heterologer Gene in Hefen im Gegensatz zu humanen oder anderen Säugetier-Zellsystemen einfacher zu handhaben und kostengünstiger.

Hefe-Expressionssysteme werden in allgemeiner Form beschrieben in Kingsman et al. (1985), "Heterologous gene expression in Saccharomyces cerevisiae", in: Biotechnology and Genetic Engineering Reviews, Vol. 3, pp 377-416.

Somit betrifft die Erfindung rekombinante Hefe-Expressionsvektoren, die die folgenden Merkmale aufweisen:
(a) eine in Hefe replizierbare DNA-Sequenz;
(b) einen Hefe-Promotor;
(c) eine DNA-Sequenz, die ein Protein mit der Immun reaktivität des HBeAg codiert;
(d) ein Transkriptions-Stop-Signal; und
(e) eine DNA-Sequenz, die einen Hefe-Selektionsmarker codiert,
wobei die DNA-Sequenz (c) die Aminosäuresequenz von der Position -29 der pre C-Sequenz bis zur Position +149 der C-Sequenz kodiert.

Der Begriff "in Hefe replizierbare DNA-Sequenz" bezeichnet einen Hefe-Replikationsursprung, aufgrund dessen die erfindungsgemäßen rekombinanten Hefe-Expressionsvektoren in Hefe-Zellen vermehrt werden und somit erhalten bleiben können. Der Begriff "Protein mit der Immunreaktivität des HBeAg" betrifft sowohl das gesamte HBeAg als auch Derivate davon, die im Vergleich zum gesamten HBeAg Aminosäure-Additionen, -Insertionen oder -Deletionen aufweisen können.

Der Fachmann kann solche Derivate des HBeAg in üblicher Weise, wie nachstehend beschrieben, auf ihre Immunreaktivität in einfachen orientierenden Versuchen testen. Ferner lassen sich immunreaktive Epitope des HBeAg mit üblichen Computerprogrammen ermitteln.

Die DNA Sequenz (c) umfaßt eine für ein Protein mit der Immunreaktivität des HBeAg codierende DNA Sequenz.

Der Begriff "Transkriptions-Stop-Signal" bezeichnet eine DNA-Sequenz, die auf die ein Protein mit der Immunreaktivität des HBeAg codierende DNA-Sequenz folgt und für eine geeignete Beendigung des Transkriptions-Vorgangs dieser codierenden DNA-Sequenz sorgt. Der Begriff "DNA-Sequenz, die einen Hefe-Selektionsmarker codiert" betrifft DNA-Sequenzen, aufgrund deren Hefen, die einen erfindungsgemäßen rekombinanten Hefe-Expressionsvektor enthalten, von den entsprechenden Plasmid-freien Hefen unterschieden werden können. Üblicherweise handelt es sich bei derartigen Selektionsmarkern für Hefe beispielsweise um Gene, die genetische Defekte der Wirtszelle komplementieren.

In einer bevorzugten Ausführungsform ist der erfindungsgemäße rekombinante Hefe-Expressionsvektor ein "shuttle"-Vektor, der nicht nur in Hefe-Wirtszellen, sondern auch in bakteriellen Wirtszellen vermehrt werden und somit erhalten bleiben kann. Dies erleichtert die Konstruktion der erfindungsgemäßen rekombinanten Hefe-Expressionsvektoren, da zur Clonierung Zwischenschritte in einfach zu handhabenden bakteriellen Wirtsorganismen durchgeführt werden können. Diese erfindungsgemäßen rekombinanten Hefe-Expressionsvektoren weisen somit die folgenden zusätzlichen Merkmale auf:
(f) einen Replikationsursprung für Bakterien; und
(g) eine DNA-Sequenz, die einen bakteriellen Selektionsmarker codiert.
Erfindungsgemäß bevorzugte "shuttle"-Vektoren sind in E. coli vermehrbar.

In einer weiteren Ausführungsform codiert die in den erfindungsgemäßen Hefe-Expressionsvektoren enthaltene DNA-Sequenz (c) am N-Terminus dieser Aminosäure-Sequenz das Signalpeptid des Killertoxins von Kluyveromyces lactis oder einen biologisch aktiven Teil dieses Signalpeptids. Vorgenanntes Signalpeptid hat nicht die mit dem Signalpeptid von alpha-Faktor verbundenen Nachteile. Als bevorzugte Ausführungsform wird eine modifizierte Signalsequenz des k.lactis Killertoxins verwendet, wobei die carboxyterminal gelegenen letzten 13 Aminosäuren der "pre-pro"-Sequenz ersetzt worden sind durch die folgenden 5 Aminosäuren Thr-Arg-Val-Lys-Arg. Das Dipeptid Lys-Arg fungiert sowohl in der Signalsequenz des Killertoxins als auch des alpha-Faktors als proteolytische Spaltstelle, so daß die neu geschaffene Hybridsignalsequenz zwei präsump tive Protease-Erkennungsstellen trägt.

Die in den Beispielen aufgeführten Expressionsvektoren enthalten HBeAG-spezifische DNA-Sequenzen des Stammes ayw, wie sie von Galibert et al. in Nature 281 (1979), 646-650, beschrieben worden sind, wobei aber die anderen HBV-Virustypen mitumfaßt sein sollen.

Nachfolgend wird der Teil der DNA-Sequenz (c) angegeben, der die Aminosäuresequenz von Ser -10 bis Val +149 des HBeAg codiert:
Erfindungsgemäß besonders bevorzugt wird eine DNA-Sequenz (c), welche die nachfolgend angegebene DNA-Sequenz enthält.

-10
TCC AAG CTG TGC CTT GGG TGG CTT TGG GGC
ser lys leu cys leu gly trp leu trp gly
ATG GAt ATC GAt CCT TAT AAA GAA TTc GGA GCT
met asp ile asp pro tyr lys glu phe gly ala

ACT GTG GAG TTA CTC TCG TTT cTc CCg agT
thr val glu leu leu ser phe leu pro ser
GAC TTC TTT CCT TCA GTA CGA GAT CTT CTg
asp phe phe pro ser val arg asp leu leu

GAT ACC GCC agc GCg CTG TAT CGG GAA GCC
asp thr ala ser ala leu tyr arg glu ala
TTg GAG TCT CCT GAG CAc TGc agc CCT CAC
leu glu ser pro glu his cys ser pro his

CAT ACT GCc CTC AGG CAA GCA ATT CTT TGC
his thr ala leu arg gln ala ile leu cys
TCG GGG GAg CTc ATG ACT CTg GCc ACg TGG
trp gly glu leu met thr leu ala thr trp

GTG GGT GTT AAc TTG GAG GAT CCT GCt TCT
val gly val asn leu glu asp pro ala ser
AGA GAC CTg GTA GTC AGT TAT GTC AAC ACT
arg asp leu val val ser tyr val asn thr

AAT ATG GGt tTA AAG TTC AGG CAA CTC TTG
asn met gly leu lys phe arg gln leu leu
TGG TTT CAC ATT agc TGc CTC ACT TTc GGc
trp phe his ile ser cys leu thr phe gly

cGA GAA ACA GTT ATA GAa TAT TTG GTG TCT
arg glu thr val ile glu tyr leu val ser
TTC GGA GTG TGG ATc AGA ACT CCT CCA GCT
phe gly val trp ile arg thr pro pro ala

TAT AGg CCt CCg AAT GCC CCT ATC CTg TCg
tyr arg pro pro asn ala pro ile leu ser
ACA CTc CCG GAG ACT ACT GTT Gtt
thr leu pro glu thr thr val val
+149

In einer anderen erfindungsgemäß besonders bevorzugten Ausführungsform hat das Signalpeptid des Killertoxins von Kluyveromyces lactis die folgende Aminosäure-Sequenz: 
Met-Asn-Ile-Phe-Tyr-Ile-Phe-Leu-Phe-Leu-Leu-Ser-Phe-Val-
Gln-Gly
 In einer anderen erfindungsgemäßen Ausführungsform des rekombinanten Hefe-Expressionsvektors ist die DNA-Sequenz (c) eine DNA-Sequenz, die mit einer der vorstehend angegebenen DNA-Sequenzen (c) hybridisiert und ein Protein mit der Immunreaktivität von HBeAg codiert. In diesem Zusammenhang bedeutet der Begriff "hybridisieren" vorzugsweise eine Hybridisierung bei Hybridisierungs-Bedingungen, bei denen der Tm-Wert zwischen Tm-20 und Tm-27 liegt. Vorzugsweise betrifft der Begriff "hybridi sieren" eine Hybridisierung unter stringenten Hybridisierungs-Bedingungen. Beispiele solcher hybridisierenden, Proteine mit der Immunreaktivität von HBeAg codierenden DNA-Sequenzen sind DNA-Sequenzen anderer HBV-Serotypen.

In einer bevorzugten Ausführungsform der erfindungsgemäßen rekombinanten Hefe-Expressionsvektoren ist die in Hefe replizierbare DNA-Sequenz die 2µ- oder ars-DNA-Sequenz.

In einer anderen bevorzugten Ausführungsform der erfindungsgemäßen rekombinanten Hefe-Expressionsvektoren ist der Hefe-Promotor der ADH-2-, GAL- oder CYC1-Promotor oder ein daraus zusammengesetzter hybrider Promotor.

In einer weiteren erfindungsgemäß bevorzugten Ausführungs-form ist die DNA-Sequenz (e) eine Aminosäuredefizienz-komplementierende DNA-Sequenz, vorzugsweise das LEU2- oder URA3-Gen.

Die Erfindung betrifft ferner Hefen, die einen der erfindungsgemäßen rekombinanten Hefe-Expressionsvektoren enthalten.

Vorzugsweise gehören diese Hefen zur Gattung Schizosaccharomyces oder Saccharomyces. Besonders bevorzugt werden Hefen der Art Schizosaccharomyces pombe oder Saccharomyces cerevisiae.

In einer anderen Ausführungsform betrifft die Erfindung Verfahren zur Herstellung eines Proteins mit der Immunreaktivität des HBeAg, bei dem man eine erfindungsgemäße Hefe, die einen erfindungsgemäßen Hefe-Expressionsvektor enthält, unter geeigneten Bedingungen züchtet und sodann das Protein mit der Immunreaktivität des HBeAg aus der Kultur isoliert.

Die Erfindung betrifft ferner nach dem erfindungsgemäßen Verfahren erhältliche Proteine mit der Immunreaktivität des HBeAg. Dieses erfindungsgemäß in Hefen hergestellte rekombinante HBeAg zeigt eine hervorragende HBeAg-Reaktivität ohne HBc-Reaktivität, ohne daß ein Denaturierungsschritt erforderlich ist. Dadurch können die erfindungsgemäßen Proteine mit der Immunreaktivität des HBeAg für hochspezifische immunologische Tests oder für eine Immunisierung verwendet werden.

In einer anderen Ausführungsform betrifft die Erfindung ein diagnostisches Reagenz, das ein erfindungsgemäßes Protein mit der Immunreaktivität des HBeAg enthält.

Gegebenenfalls ist das erfindungsgemäße Protein mit der Immunreaktivität des HBeAg dabei mit einer nachweisbaren Markierung, vorzugsweise mit einer radioaktiven, enzymatischen, fluoreszierenden oder chemolumineszenten Markierung verknüpft. Bei diesen Markierungen handelt es sich um übliche Markierungen, beispielsweise um die Markierung mit Jod-125, Meerrettich-Peroxidase, *β*-Galaktosidase, Fluoreszein oder Acridiniumester.

In einer bevorzugten Ausführungsform wird das erfindungsgemäße diagnostische Reagenz zur Durchführung von Immunoassays zum Nachweis von HBeAg oder Anti-HBeAg-Antikörpern verwendet. Beispiele solcher Immunoassays sind die bereits erwähnten kommerziell erhältlichen Assays, sowie die in den Beispielen aufgeführten Assays.

Die Erfindung betrifft ferner Arzneimittel, die ein erfindungsgemäßes Protein mit der Immunreaktivität des HBeAg gegebenenfalls in Kombination mit einem pharmazeutisch verträglichen Träger und/oder Verdünnungsmittel enthalten. Gegebenenfalls können in diesen Arzneimitteln weitere Immunogene, wie HBsAg/HBcAg/preS einzeln, in Mischung oder als Fusionsprotein enthalten sein. Somit enthalten die erfindungsgemäßen Arzneimittel das HBeAg als Impfstoff alleine; das HBeAg als Impfstoff in einer Mischung mit verschiedenen anderen Hepatitis B-Antigenen, wie HBsAg und/oder HBcAg; das HBeAg als Impfstoff mit anderen Hepatitis B-Antigenen als Fusionsprotein; das HBeAg als Mischung mit ganz anderen Impfstoffen (Kombinationsimpfung oder als Verstärker der Impfantwort); oder das HBeAg als Fusionsprotein mit einem ganz anderen Protein (z.B. von HIV) für die Kombinationsimpfung oder als Verstärker der Immunantwort.

Schließlich betrifft die Erfindung Verfahren zur Immunisierung von Menschen gegen HBV-Infektionen, bei denen ein erfindungsgemäßes Protein mit der Immunreaktivität des HBeAg gegebenenfalls in Kombination mit einem pharmazeutisch verträglichen Träger und/oder Verdünnungsmittel in üblicher Weise verimpft wird.

Vorzugsweise handelt es sich bei den erfindungsgemäßen Arzneimitteln um Hepatitis-Impfstoffe.

Die erfindungsgemäßen Proteine mit der Immunreaktivität des HBeAg lassen sich ferner in hochspezifischer Weise zur Herstellung von polyclonalen oder monoklonalen anti-HBeAg-Antikörpern verwenden. Dabei werden Säugetiere, vorzugsweise Nager, wie Kaninchen, Meerschweinchen, Ratten oder Mäuse oder auch Huftiere, wie Schafe und Pferde, mit dem erfindungsgemäßen Protein immunisiert, und sodann werden die Antikörper aus dem Blutserum isoliert. Die Herstellung von monoklonalen Antikörpern erfolgt nach den üblichen Verfahren.

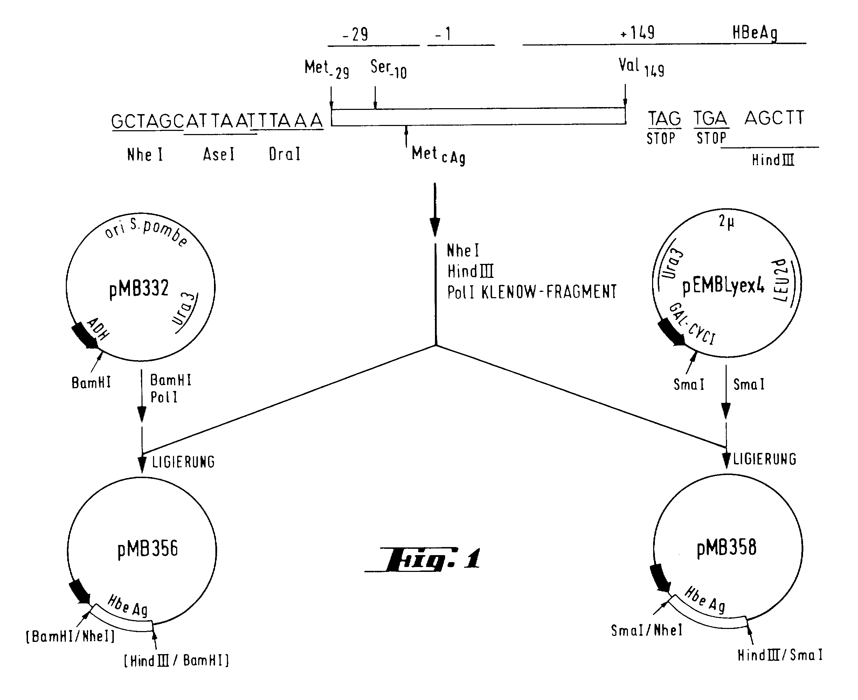

Um HBeAg in Hefen exprimieren zu können, wurde in einer bevorzugten Ausführungsform eine DNA-Sequenz konstruiert, die den codierenden Abschnitt vom Codon der Aminosäure -29 des HBcAg-Gens bis zum Codon der Aminosäure +149 trägt. Am 5'-Terminus wurde eine DNA-Sequenz angefügt, die Schnittstellen für einige Restriktionsendonucleasen enthält und in Position -3 vor dem HBeAg-Translationsstart ein A aufweist, damit eine effiziente Translation der rekombinanten mRNA in Hefen gewährleistet ist. Am 3'-Terminus (der die Aminosäure +149 codiert) folgen zwei Stopcodons und eine HindIII-Clonierungsstelle (Figur 1). Dieses DNA-Fragment wurde in Expressionsvektoren für S. pombe und S. cerevisiae cloniert und die rekombinanten Plasmide in geeignete Stämme transformiert, die nun HBeAg exprimieren können.

Die nachfolgenden Figuren und Beispiele betreffen teilweise Gegenstände, die nicht mehr beansprucht werden. Diese erläutern jedoch die Erfindung.

Die vorliegende Erfindung wird außerdem-durch die Patentansprüche beschrieben.

Die Figuren zeigen:
- Figur 1:: Konstruktion von Expressionsvektoren die die Synthese von HBeAg von Met-29 bis Val+149 in Saccharomyces cerevisiae und Schizosaccharomyces pombe codieren.
- Figur 2:: Konstruktion eines Expressionsvektors, der die Synthese von HBeAg von Ser-10 bis Val+149 in Saccharomyces cerevisiae codiert.
- Figure 3:: Konstruktion eines Expressionsvektors, der die Synthese von HBeAg von Ser-10 bis Val+149 fusioniert an eine Hefe-Signalsequenz codiert.

Die Beispiele erläutern die Erfindung. Sie beschreiben unter anderem die Expression von HBeAg in der Spalthefe Schizosaccharomyces pombe und in der Bäckerhefe Saccharomyces cerevisiae. Weitere Informationen zu den angewendeten molekularbiologischen Methoden sind beschrieben in Sambrook et al., "Molecular Cloning", 2. Auflage, Cold Spring Harbour, 1989.

### Beispiel 1

### Konstruktion eines Vektors zur Expression von HBeAg in S. pombe

Das NheI/HindIII-Fragment, das für das HBeAg codiert, wurde mit PolymeraseI Klenow-Fragment in Gegenwart von Nucleotiden behandelt, um die überstehenden Einzelstränge zu reparieren. Das DNA-Fragment wurde in die singuläre BamHI-Stelle des S. pombe-Expressionsvektors pMB332 (Bröker und Bäuml, FEBS Lett. 248 (1989), 105 - 110), die ebenfalls mit Poll behandelt worden war, ligiert. Im neuen Plasmid pMB356 steht somit die Expression des HBeAg unter der Kontrolle des S. pombe-ADH-Promotors. S. pombe ura4-Stämme können mit diesem Plasmid durch das auf dem Vektor pMB356 befindliche S. cerevisiae-URA3-Gen komplementiert und auf Minimalmedium selektioniert werden.

### Beispiel 2

### Konstruktion eines Vektors zur Expression von HBeAg in S. cerevisiae

Dasselbe HBeAg-codierende DNA-Fragment wie in Beispiel 1 wurde in die singuläre SmaI-Stelle des S. cerevisiae-Expressionsvektors pEMBLyex4 (Cesareni and Murray, "Genetic Engineering, Setlow, ed., Vol. 9 (1987), S. 135 - 153) ligiert. Im neuen Plasmid pMB358 steht die Expression des HBeAg somit unter der Kontrolle des regulierbaren GAL-CYC1-Hybridpromotors. S. cerevisiae ura3- und/oder leu2-Stämme können mit dem Vektor pMB358 zur Uracil- bzw. Leucin-Prototrophie durch die auf dem Vektor befindlichen LEV2- und URA3-Gene komplementiert werden.

### Beispiel 3

### Expression des HBeAg in Hefe

Die Plasmide pMB356 bzw. 358 wurden in S. pombe ura4 bzw. S. cerevisiae (z. B. Stamm C13ABYS86, leu2, ura3, his) mit Hilfe der LiCl-Methode (Bröker, Biotechniques 5 (1987), 516 - 518) transformiert und Transformanten auf YNB-Medium selektioniert.

Vorkulturen mit je 50 ml YNB-Medium in 300 ml Erlenmeyerkolben wurden mit Einzelkolonien angeimpft und 48 h bei 30 °C geschüttelt. Anschließend wurden 100 ml YPD-Medium mit 10 ml dieser Vorkultur überimpft. Die Induktion des GAL-CYC1-Promotors erfolgte durch Zugabe von 2 % Galactose zur Kultur. Die Ausbeute in HBeAg in S. cerevisiae kann durch Wachstumsbedingungen, wie sie in der DE-PS 39 40 651 beschrieben sind, wesentlich erhöht werden.

Die Zellen wurden nach drei bis vier Tagen geerntet und mit Glaskugeln aufgeschlossen. Der lösliche Überstand enthielt immunoreaktives HBeAg. Vorzugsweise wurde folgender Puffer für den Zellaufschluß gewählt: 0,38 % Na-Citrat, pH 7,2; 0,85 % NaCl unter Zusatz von Benzamidinchlorid (2 mg/ml), Phenymethylsulfonylfluorid (1mM), Polybren (1 mg/ml) und Antagosan (100 KIE/ml). In diesem Puffer ist das rekombinante HBeAg ohne Reaktivitätsverlust bei -70 °C lagerfähig.

### Beispiel 4

### Konstruktion eines Vektors zur Expression von rHBeAg Ser -10 bis Val +149 in S. cerevisiae

Kim et al.,a.a.O., haben beschrieben, daß in Hefen synthetisiertes HBeAg ohne die Präcore-Sequenz und ohne den Arginin-reichen C-Terminus keine HBeAg-Reaktivität hat, sondern HBcAg-Reaktivität aufweist. Es wurde deshalb ein Expressionsvektor entwickelt, der im Gegensatz zu den Befunden von Kim et al. die Direktexpression von "prozessiertem HBeAg" gewährleistet und ein Polypeptid ohne HBcAg-Reaktivität liefert.

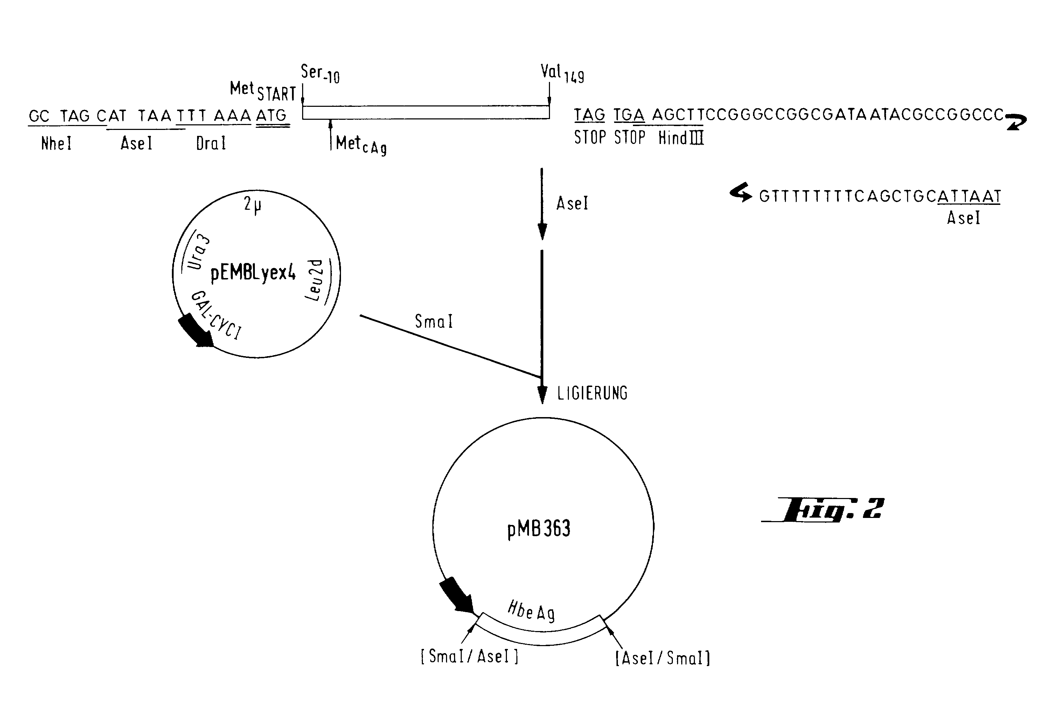

In Analogie zu Beispiel 2 wurde ein DNA-Fragment, daß für HBeAg von Ser -10 bis Val +149 codiert in den Vektor pEMBLyex4 cloniert (Figur 2). Da diese modifizierte HBeAg DNA im Plasmid pMB363 kein eigenes Translationsstartcodon beinhaltet, wurde der zu translatierenden Sequenz ein ATG-Triplett vorgeschaltet, so daß das rekombinante HBeAg mit Met-Ser -10 am Aminoterminus beginnt. Im folgenden wird dieses Material als rHBeAg Ser -10 bis Val +149 bezeichnet.

### Beispiel 5

### Konstruktion eines Vektors zur Expression von rHBeAg Ss:: Ser -10 bis Val +149 fusioniert an die Signalsequenz des Killertoxins von Kluyveromyces lactis

Kim et al., a.a.O., haben postuliert, daß in Hefen synthetisiertes HBeAg durch eine Signalsequenz transloziert werden muß, um HBeAg-Reaktivität zu erhalten. Sie haben dazu eine Fusionierung der codierenden DNA-Sequenz des HBeAg an die Signalsequenz des S. cerevisiae α-Faktors vorgenommen.

Nachfolgend wird gezeigt, daß man auch eine Fusionierung an die Signalsequenz des Kluyveromyces lactis-Killertoxins vornehmen kann.

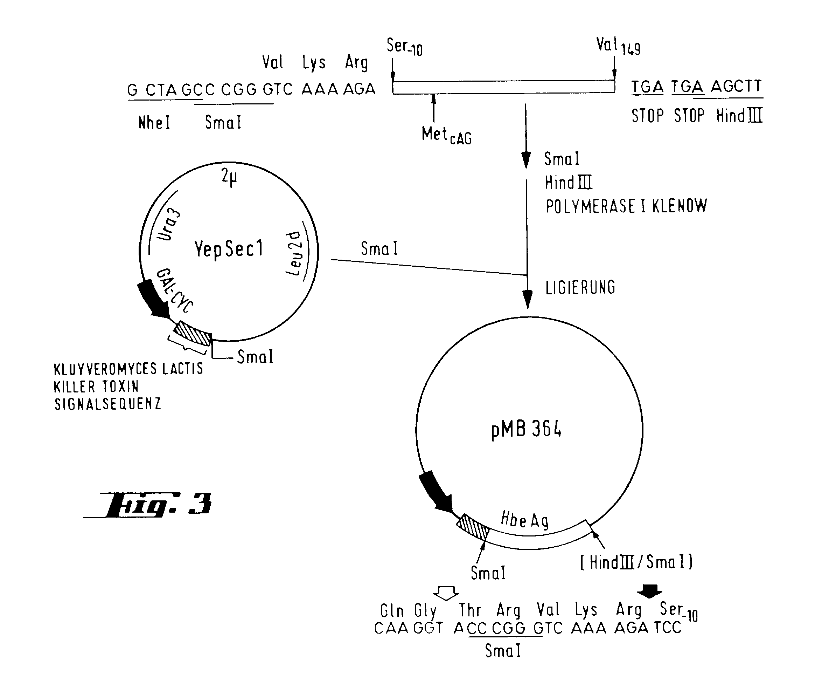

Das HBeAg, wie es durch den Vektor pMB363 codiert ist (Beispiel 4), sollte nicht sezerniert werden, sondern im Cytoplasma der Hefezellen verbleiben. Durch Fusionierung der HBeAg-DNA an eine Signalsequenz eines sekretorischen Hefeproteins sollte es eventuell möglich sein, das rekombinante HBeAg zu sezernieren. Dazu wurde, wie in Figur 3 dargestellt, das Plasmid pMB364 konstruiert. Durch Fusionierung an die Signalsequenz des Killertoxins von K. lactis, codiert durch den Vektor YEpsec1 (Baldari et al., EMBO J. 6 (1987), 229 - 234) ergeben sich zwei mögliche spezifische Spaltstellen: die postulierte des Killertoxins und fünf Aminosäuren weiter die des alpha-Faktors von Saccharomyces cerevisiae. Bei oder während der Translokation des Fusionsproteins könnte eine oder beide dieser Spaltstellen genutzt und das prozessierte HBeAg sezerniert werden.

In der Kulturbrühe von S. cerevisiae (pMB364) konnte kein Antigen mit HBeAg-Reaktivität nachgewiesen werden, wohl aber in Zellextrakten der rekombinanten Hefen. Im folgenden wird dieses Material als rHBeAg Ss:: Ser -10 bis Val +149 bezeichnet.

### Beispiel 6

### Spezielle Assays für HBeAg und HBcAg

Die kommerziell erhältlichen Testsysteme HBe (rDNA) (Abbott, Wiesbaden) und Enzygnost HBe (Behringwerke, Marburg) erkennen neben HBeAg auch HBcAg, da die verwendeten Festphasen und Konjugate unter Verwendung von Antikörperpräparationen von HBV-infizierten Menschen hergestellt wurden und somit neben den Anti-HBeAg-Antikörpern auch Anti-HBcAg-Antikörper enthalten. Um Präparationen mit HBeAg- und/oder HBcAg-Gehalt spezifisch auf das jeweilige Antigen testen zu können, sind spezielle Immunoassays erforderlich. Wir haben deshalb einen spezifischen Assay zur Bestimmung des HBcAg mittels eines monoklonalen Antikörpers und einen Assay zur spezifischen Bestimmung des HBeAg mittels monoklonaler Antikörper etabliert.

### Immunoassay für HBcAg

In die mit einem monoklonalen Anti-HBcAg-Antikörper beschichteten Näpfchen einer Mikrotitrationsplatte werden 100 *µ*l der jeweiligen Probe 1 h bei 37 °C inkubiert. Anschliessend wird 2 x gewaschen und dann 1 h bei 37 °C mit einem monoklonalen Anti-HBcAg/POD-Konjugat (M. Noah, H.-P. Harthus, Bio Engineering 4 (1988), 22 - 30; Salfeld et al., J. Virol. 63 (1989), 798 - 808) inkubiert. Nach einem erneuten Waschgang erfolgt die Zugabe von 100 *µ*l Chromogen/Substratlösung (Tetramethylbenzidin, siehe Enzygnost HIV 1+2, Behringwerke, Marburg). Die Chromogenreaktion wird nach 30 Minuten durch die Zugabe von 100 *µ*l 0,5nH₂SO₄ gestoppt.

Die Extinktionsmessung erfolgt bei einer Wellenlänge von 450 nm. Als HBcAg-positiv werden alle Proben betrachtet, deren Extinktion mehr als 0,05 E über dem Mittelwert der negativen Kontrollen liegt.

### Immunoassay für HBeAg (monoklonal)

Der Immunoassay für HBeAg folgt dem gleichen Schema wie der Assay für das HBcAg; nur werden Mikrotitrationsplatten verwendet, die mit monoklonalen Anti-HBeAg-Antikörpern beschichtet sind und ein Konjugat aus monoklonalen Anti-HBeAg-Antikörpern. Die Erzeugung monoklonaler Anti-HBeAg-Antikörper ist in der Literatur mehrfach beschrieben (M. Imai et al., J. of Immunol. 128 (1982), 69 - 75; R.B. Ferns, R.S. Tedder, J. Gen. Virol. 65 (1984), 899 - 908). Auch der kommerzielle HBeAg-Test der Firma Sorin verwendet bereits monoklonale Antikörper. Als HBeAg-positiv werden Proben betrachtet, deren Extinktion mehr als 0,05 E über dem Mittelwert der negativen Kontrollen liegt.

### Beispiel 7

### Sensitivität und Spezifität des Enzygnost HBe, des monoklonalen HBeAg- und des monoklonalen HBcAg-ELISA's

In den Testsystemen Enzygnost HBe, HBeAg monoklonal und HBcAg monoklonal wurden verschiedene Materalien eingesetzt, um die Sensitivität und Spezifität der Teste aufzuzeigen und um die HBeAg- und/oder HBcAg-Reaktivität dieser Materialien zu demonstrieren. Bei diesen Materialien handelte es sich um ein rekombinantes Material aus E.coli, welches die Aminosäuren bis +183 der C-Sequenz umfaßte (r183 / Biogen, Cambridge, Massachusetts, USA) und um das gleiche Material nach SDS-Behandlung (r183 SDS), um die HBcAg-Reaktivität zu senken und die HBeAg-Reaktivität zu induzieren bzw. zu erhöhen (R.B. Ferns, R.S. Tedder, a.a.O).

Weiterhin wurde ein verkürztes, rekombinantes Material aus E.coli verwendet, das nur den Aminosäuren bis +144 der C-Sequenz entsprach (r144, Biogen) sowie natives HBeAg aus Human-Serum, dessen Gehalt am Standardmaterial des Paul-Ehrlich-Instituts kalibriert war. Da HBeAg-haltiges Humanserum immer Anti-HBcAg-Antikörper enthalten muß, eignet sich dieses Material hervorragend, um die analytische Sensitivität zweier Testsysteme in Bezug auf HBeAg-Reaktivität zu vergleichen, wenn ein Test neben HBeAg auch HBcAg erkennt. Für die Verwendung im Test auf HBcAg ist dieses Material ungeeignet, da die Anti-HBcAg-Antikörper eine eventuell vorhandene HBcAg-Reaktivität vollkommen maskieren würden. Der Vollständigkeit wegen sind in der Tabelle 1 aber auch diese Daten mit angegeben.

Vergleicht man insbesondere die Ergebnisse des Enzygnost HBe mit dem Test HBeAg monoklonal, zeigt sich in Tabelle 1 A die höhere Sensitivität für HBeAg im monoklonalen Test.

Beim r183 hingegen erscheint der Enzygnost HBe etwa 20 x sensitiver, was auf den Gehalt an HBcAg- und HBeAg-Reaktivität in diesem Material und die zusätzliche Spezifität des Enzygnost-HBe auch für HBcAg zurückzuführen ist.

Vergleicht man jetzt das rekombinante Material r183 im HBcAg monoklonal und im HBeAg monoklonal, zeigt sich eine mindestens 7fach stärker ausgeprägte HBcAg- als HBeAg-Reaktivität dieses Materials (Tabelle 1 B). Durch die SDS-Behandlung sinkt die HBcAg-Reaktivität auf weniger als 1/100 ab, die HBeAg-Reaktivität nur etwa auf 1/5. Das heißt, daß die HBcAg-Reaktivität immer noch etwa 1/300 der HBeAg-Reaktivität beträgt (Tabelle 1B und 1C).

Das rekombinante Material r144 weist etwa 1/6 der HBeAg-Reaktivität als HBcAg-Reaktivität auf (Tabelle 1 D).

Hingegen weist das erfindungsgemäße rekombinante Hefe-Material (Beispiel 3) ohne weitere Behandlung als HBcAg-Reaktivität weniger als 1/3000 der HBeAg-Reaktivität auf (Tabelle 1 E); zur weiteren Abschätzung der HBcAg-Kreuzreaktivität dieses Materials siehe Beispiel 9.

Zusammengefaßt zeigt die Tabelle 1, daß
a) Enzygnost^{R}-HBe sowohl HBeAg als auch HBcAg zu erkennen vermag;
b) Enzygnost^{R}-HBe monoklonal spezifisch ist für HBeAg und mehr als doppelt so sensitiv ist wie der Enzygnost^{R}-HBe;
c) der Test HBc monoklonal spezifisch ist für HBcAg;
d) r183 sowohl HBeAg- als auch HBcAg-Reaktivität aufweist und durch SDS-Behandlung zum Teil HBcAg-Reaktivität und HBeAg-Reaktivität verliert;
e) r144 sowohl HBeAg- als auch deutlich HBcAg-Reaktivität aufweist;
f) das erfindungsgemäße rekombinante Hefe-HBeAg mit einer HBcAg-Aktivität von weniger als 1/3000 der HBeAg-Reaktivität praktisch frei von HBcAg-Reaktivität ist.
Diese geringen Kreuzreaktivitäten (1:10000) könnten durch den monoklonalen Anti-HBcAg-Antikörper verursacht werden.

**Tabelle 1**

| **Vergleich der Sensitivität und Spezifität des Enzygnost-HBe, des monoklonalen HBeAg und des monoklonalen HBcAg-Assays** | | | | |
|---|---|---|---|---|
| **Probe** | | **E.-HBe [mE]** | **HBeAg monokl. [mE]** | **HBcAg monokl. [mE]** |
| neg. Kontr. | | 19 | 3 | 5 |
| | | | | |
| HBeAg-Serum | 7,5 U/ml | 810 | < 2.500 | 6 |
| | 3,75 U/ml | 613 | 1.725 | 8 |
| A | 1,88 U/ml | 335 | 850 | 5 |
| | 0,94 U/ml | 179 | 522 | 6 |
| | 0,47 U/ml | 96 | 248 | 6 |
| | 0,23 U/ml | 75 | 125 | 6 |
| | 0,12 U/ml | 46 | 56 | 5 |
| analytische Sensitivität | | 0,24 E/ml | 0,11 E/ml | ./. |
| | | | | |
| r183 | 10 µg/ml | > 2.500 | > 2.500 | > 2.500 |
| | 1 µg/ml | > 2.500 | > 2.500 | > 2.500 |
| B | 100 ng/ml | > 2.500 | 366 | > 2.500 |
| | 10 ng/ml | 665 | 11 | 39 |
| | 1 ng/ml | 35 | 5 | 5 |
| | | | | |
| r183 SDS | 10 *µ*g/ml | > 2.500 | > 2.500 | 232 |
| | 1 *µ*g/ml | 445 | 1.040 | 5 |
| C | 100 ng/ml | 54 | 79 | 3. |
| | 10 ng/ml | 25 | 7 | 4 |
| | 1 ng/ml | 20 | 3 | 6 |
| | | | | |
| r144 | 1 : 100 | > 2.500 | > 2.500 | > 2.500 |
| | 1 : 1.000 | > 2.500 | > 2.500 | > 2.500 |
| D | 1 : 10.000 | > 2.500 | > 2.500 | > 2.500 |
| | 1 : 100.000 | > 2.500 | > 2.500 | > 2.500 |
| | 1 : 1.000.000 | > 2.500 | > 2.500 | 1.039 |
| | 1 : 10.000.000 | 1.020 | 1.829 | 8 |
| | 1 : 100.000.000 | 193 | 364 | 13 |
| | 1 : 1.000.000.000 | 77 | 122 | 5 |
| | | | | |
| Hefe rHBeAg SC 358-3004 | | | | |
| | unverdünnt | < 2.500 | < 2.500 | 6 |
| | 1 : 10 | 1.263 | < 2.500 | 8 |
| E | 1 : 100 | 151 | 439 | 5 |
| | 1 : 1.000 | 32 | 28 | 6 |
| | 1 : 10.000 | 23 | n.d. | 4 |

### Beispiel 8

### Immunreaktivität des HBeAg aus S. pombe

Die HBeAg-haltigen löslichen Zellextrakte individueller-Transformationen von S. pombe laut Beispiel 3 wurden in den verschiedenen Immunoassays auf HBeAg- und HBcAg-Reaktivität getestet. Es wurden vier individuelle Transformationen (A - D) in YNB-Medium herangezogen und der lösliche Zellextrakt untersucht.

Wie aus Tabelle 2 ersichtlich ist, weisen die Clone YNB A, YNB B, YNB C und YNB D gute HBeAg-Reaktivität auf, ohne daß eine HBcAg-Reaktivität auch nur angedeutet wäre.

**Tabelle 2**

| **Immunreaktivität des HBeAg aus S. pombe** | | | | |
|---|---|---|---|---|
| **Probe** | | **Enz. HBe [mE]** | **HBeAg monokl. [mE]** | **HBcAg monokl. [mE]** |
| neg. Kontrolle | | 25 | 17 | 17 |
| | | | | |
| Clon YNB A | unverd. | 225 | 606 | 11 |
| | 1 : 10 | 29 | 60 | 8 |
| | | | | |
| Clon YNB B | unverd. | 141 | 451 | 14 |
| | 1 : 10 | 31 | 40 | 8 |
| | | | | |
| Clon YNB C | unverd. | 938 | 1924 | 11 |
| | 1 : 10 | 71 | 226 | 11 |
| | | | | |
| Clon YNB D | unverd. | 580 | 1263 | 15 |
| | 1 : 10 | 98 | 298 | 9 |

### Beispiel 9

### Immunreaktivität des HBeAg aus S. cerevisiae

Die HBeAg-haltigen löslichen Zellextrakte individueller Transformationen von S. cerevisiae laut Beispiel 3 wurden in verschiedenen Immunoassays auf HBeAg- und HBcAg-Reaktivität getestet.

Es wurden 5 individuelle Transformationen (SC-A bis SC-E) herangezogen und der lösliche Zellextrakt untersucht.

Die Überstände zeigten eine sehr hohe HBeAg-Reaktivität, wobei die HBcAg-Reaktivität nur etwa 1 : 10.000 der HBeAg-Reaktivität ausmacht.

**Tabelle 3**

| **Immunreaktivität des HBeAg aus S. cerevisiae** | | | | |
|---|---|---|---|---|
| **Probe** | | **Enz.HBe [mE]** | **HBeAg monokl. [mE]** | **HBcAg monokl. [mE]** |
| neg. Kontr. | | 22 | 9 | 15 |
| rHBcAg 100 ng/ml | | > 2.500 | 770 | > 2.500 |
| | | | | |
| SC-A | unverd. | > 2.500 | > 2.500 | 34 |
| | 1 : 10 | > 2.500 | > 2.500 | 19 |
| | 1 : 100 | n.d. | > 2.500 | n.d. |
| | 1 : 1.000 | n.d. | 761 | n.d. |
| | 1 : 10.000 | n.d. | 51 | n.d. |
| | | | | |
| SC-B | unverd. | > 2.500 | > 2.500 | 35 |
| | 1 : 10 | > 2.500 | > 2.500 | 15 |
| | 1 : 100 | n.d. | > 2.500 | n.d. |
| | 1 : 1.000 | n.d. | 297 | n.d. |
| | 1 : 10.000 | n.d. | 23 | n.d. |
| | | | | |
| SC-C | unverd. | > 2.500 | > 2.500 | 67 |
| | 1 : 10 | > 2.500 | > 2.500 | 21 |
| | 1 : 100 | n.d. | > 2.500 | n.d. |
| | 1 : 1.000 | n.d. | 626. | n.d. |
| | 1 : 10.000 | n.d. | 57 | n.d. |
| | | | | |
| SC-D | unverd. | > 2.500 | > 2.500 | 32 |
| | 1 : 10 | > 2.500 | > 2.500 | 17 |
| | 1 : 100 | n.d. | > 2.500 | n.d. |
| | 1 : 1.000 | n.d. | 585 | n.d. |
| | 1 : 10.000 | n.d. | 48 | n.d. |
| | | | | |
| SC-E | unverd. | > 2.500 | > 2.500 | 32 |
| | 1 : 10 | > 2.500 | > 2.500 | 17 |
| | 1 : 100 | n.d. | > 2.500 | n.d. |
| | 1 : 1.000 | n.d. | 519 | n.d. |
| | 1 : 10.000 | n.d. | 44 | n.d. |

### Beispiel 10

### Immunoassay zur Bestimmung von Anti-HBeAg-Antikörpern aus Humanproben mittels eines konventionellen Neutralisationsreagenzes und eines Neutralisationsreagenzes aus rekombinantem HBeAg aus S.c.

### (A) Unter Verwendung eines Assays mit polyklonalen, humanen Festphasen- und Konjugatantikörpern

Der Enzygnost HBe verwendet als Festphasen und Konjugatantikörper Antikörperpräparationen aus Humanseren. Ebenso stammt das als HBeAg verwendete Material aus dem Blut infizierter Personen. Um zu zeigen, daß das rekombinante HBeAg sich für die Bestimmung von Anti-HBeAg-Antikörpern aus Humanproben ebenso eignet wie das HBeAg aus Blut, wurde der Test zum einen gemäß Packungsbeilage durchgeführt, zum anderen mit einer entsprechenden Verdünnung des rekombinanten Materials aus Beispiel 3.

Als Proben wurde Material von akut oder chronisch infizierten Personen verwendet, die weiterhin noch andere Parameter einer Hepatitis B Virus-Infektion aufwiesen. Außerdem wurde zur Ermittlung der analytischen Sensitivität ein Anti-HBeAg-Material eingesetzt, das am Standardmaterial des Paul-Ehrlich-Institutes, Frankfurt, kalibriert war.

Wie Tabellen 4 a) und b) zeigen, sind die Ergebnisse mit dem rekombinanten HBeAg absolut mit den Ergebnissen des konventionellen Materials vergleichbar.

**Tabelle 4 a)**

| **Vergleich des Enzygnost HBe mit konventionell gewonnenem HBeAg und mit rekombinantem HBeAg als Neutralisations-reagenz** | | |
|---|---|---|
| Analytische Sensitivität anhand eines am Standardmaterial des Paul-Ehrlich-Institutes, Frankfurt, kalibrierten Sekundärstandards | | |

| **Probe** | **Enzygnost-HBe** | |
|---|---|---|
| | **konv. HBeAg [mE]** | **rek. HBeAg [mE]** |
| | | |
| neg. Kontrolle | 603 | 1.094 |
| cut off | 302 | 547 |
| 0,75 E/ml | 252 | 84 |
| 0,38 E/ml | 435 | 155 |
| 0,19 E/ml | 519 | 323 |
| 0,09 E/ml | 589 | 469 |
| 0,05 E/ml | 620 | 657 |
| 0,02 E/ml | 574 | 787 |
| | | |
| analyt. Sensitivität | 0,65 U/ml | 0,08 U/ml |

**Tabelle 4 b)**

| **Vergleich des Enzygnost HBe mit konventionell gewonnenem HBeAg und mit rekombinantem HBeAg als Neutralisations-reagenz im Anti-HBe positiven Kollektiv** | | |
|---|---|---|
| **Probe** | **Enzygnost-HBe** | |
| | **konv. HBeAg [mE]** | **rek. HBeAg [mE]** |
| neg. Kontrolle | 1.276 | 1.727 |
| pos. Kontrolle | 43 | 59 |
| cut off | 638 | 864 |
| | | |
| Nr. KK 2 | 57 | 43 |
| 3 | 70 | 26 |
| 4 | 23 | 26 |
| 5 | 25 | 30 |
| 7 | 34 | 25 |
| 8 | 22 | 30 |
| 12 | 516 | 88 |
| 15 | 31 | 61 |
| 16 | 247 | 101 |
| 21 | 48 | 52 |
| 22 | 28 | 50 |
| 23 | 48 | 40 |
| 24 | 80 | 30 |
| 25 | 53 | 47 |
| 30 | 75 | 67 |
| 35 | 65 | 45 |
| 36 | 33 | 59 |
| 37 | 158 | 48 |
| 38 | 107 | 40 |
| 40 | 51 | 39 |
| 41 | 56 | 48 |
| 42 | 74 | 35 |
| 44 | 90 | 36 |
| 46 | 32 | 29 |
| 50 | 220 | 129 |

### (B) Unter Verwendung eines Assays mit monoklonalen Festphasen- und Konjugatantikörpern

Der im Beispiel 6 beschriebene monoklonale Assay für HBeAg wurde durch die Verwendung eines konventionellen Neutralisationsreagenzes bzw. eines Neutralisationsreagenzes mit rekombinantem HBeAg so modifiziert, daß Anti-HBeAg-Antikörper aus Humanproben nachgewiesen werden konnten.
Als Proben diente das gleiche Material wie unter 10 A. Wie die Tabellen 5 a) und b) zeigen, sind auch im monoklonalen Test die Ergebnisse des konventionellen und des rekombinanten Materials absolut vergleichbar.

**Tabelle 5**

| **Vergleich des HBe monoklonal mit konventionell gewonnenem HBeAg und rekombinantem HBeAg als Neutralisationsreagenz** | | |
|---|---|---|
| 5 a) Analytische Sensitivität anhand eines am Standardmaterial des Paul-Ehrlich-Instituts, Frankfurt, kalibrierten Sekundärstandards | | |
| **Probe** | **HBe monoklonal** | |
| | **konv. HBeAg [mE]** | **rek. HBeAg [mE] neg.** |
| Kontrolle | 1.508 | 1.513 |
| cut off | 754 | 757 |
| 0,75 E/ml | 142 | 112 |
| 0,38 E/ml | 375 | 279 |
| 0,19 E/ml | 723 | 549 |
| 0,09 E/ml | 985 | 769 |
| 0,05 E/ml | 1.265 | 1.022 |
| 0,02 E/ml | 1.501 | 1.207 |
| | | |
| analyt. Sensitivität | 0,18 E/ml | 0,11 E/ml |

| 5 b) im Anti-Hbe positiven Kollektiv | | |
|---|---|---|
| **Probe** | **HBe monoklonal** | |
| | **konv. HBeAg [mE]** | **rek. HBeAg [mE]** |
| neg. Kontrolle | 1.806 | 1.381 |
| pos. Kontrolle | 6 | 12 |
| cut off | 903 | 690 |
| | | |
| Nr. KK 2 | 6 | 10 |
| 3 | 8 | 3 |
| 4 | 4 | 4 |
| 5 | 3 | 13 |
| 7 | 3 | 4 |
| 8 | 3 | 31 |
| 12 | 174 | 168 |
| 15 | 3 | 7 |
| 16 | 114 | 184 |
| 21 | 5 | 18 |
| 22 | 3 | 14 |
| 23 | 3 | 6 |
| 24 | 5 | 3 |
| 25 | 3 | 4 |
| 30 | 3 | 14 |
| 35 | 5 | 8 |
| 36 | 3 | 5 |
| 37 | 45 | 45 |
| 38 | 23 | 49 |
| 40 | 11 | 4 |
| 41 | 19 | 21 |
| 42 | 30 | 6 |
| 44 | 122 | 19 |
| 46 | 4 | 4 |
| 50 | 130 | 112 |

### Beispiel 11

### Immunreaktivität des rekombinanten HBeAg Ser -10 bis Val +149 mit und ohne vorgeschaltete Signalsequenz aus K. lactis

In Tabelle 6 wird gezeigt, daß auch die Materialien aus Beispiel 4 (rHBeAg Ser -10 bis Val +149) und aus Beispiel 5 (rHBeAg Ss:: Ser -10 bis Val +149) über eine gute HBeAg-Reaktivität verfügen und keine HBcAg-Reaktivität aufweisen.

**Tabelle 6**

| **Immunreaktivität von rekombinanten HBeAg Ser -10 bis Val +149 und HBeAg Ss:: Ser -10 bis Val +149** | | | |
|---|---|---|---|
| **Probe** | **Enz. HBe [mE]** | **HBeAg monokl. [mE]** | **HBcAg monokl. [mE]** |
| neg. Kontrolle | 18 | 8 | 6 |
| | | | |
| rHBeAg Ser -10 bis Val +149 | | | |
| 1 : 10 | 1.780 | > 2.500 | 7 |
| 1 : 100 | 185 | 1.755 | 6 |
| 1 : 1.000 | 43 | 188 | 5 |
| 1 : 10.000 | 20 | 29 | 6 |
| | | | |
| rHBeAg Ss:: Ser -10 bis Val +149 | | | |
| 1 : 10 | > 2.500 | > 2.500 | 8 |
| 1 : 100 | 1.098 | > 2.500 | 7 |
| 1 : 1.000 | 118 | 1.077 | 7 |
| 1 : 10.000 | 35 | 147 | 6 |

## Patentansprüche (Patentansprüche für folgende(n) Vertragsstaat(en): BE, CH, DE, DK, FR, GB, IT, Ll, LU, NL, PT, SE)

1. Rekombinanter Hefe-Expressionsvektor, der die folgenden Merkmale aufweist:
(a) eine in Hefe replizierbare DNA-Sequenz;
(b) einen Hefe-Promotor;
(c) eine DNA-Sequenz, die ein Protein mit der Immunreaktivität des HBeAg kodiert;
(d) ein Transkriptions-Stop-Signal; und
(e) eine DNA-Sequenz, die einen Hefe-Selektionsmarker kodiert,
dadurch gekennzeichnet, daß die DNA-Sequenz (c) die Aminosäuresequenz von der Position -29 der pre C-Sequenz bis zur Position +149 der C-Sequenz kodiert.

2. Rekombinanter Hefe-Expressionsvektor nach Anspruch 1, der außerdem die folgenden Merkmale aufweist:
(f) einen Replikationsursprung für Bakterien; und
(g) eine DNA-Sequenz, die einen bakteriellen Selektionsmarker kodiert.

3. Rekombinanter Hefe-Expressionsvektor nach Anspruch 1, dadurch gekennzeichnet, daß er zusätzlich das Signalpeptid des Killertoxins von Kluyveromyces lactis oder einen biologisch aktiven Teil dieses Signalpeptids enthält.

4. Rekombinanter Hefe-Expressionsvektor nach Anspruch 3, wobei das Signalpeptid des Killertoxins von Kluyveromyces lactis die folgende Aminosäure-Sequenz aufweist:

Met-Asn-Ile-Phe-Tyr-Ile-Phe-Leu-Phe-Leu-Leu-Ser-Phe-Val-Gln-Gly

5. Rekombinanter Hefe-Expressionsvektor nach Anspruch 1, wobei die DNA-Sequenz (c) eine DNA-Sequenz ist, die mit einer in Anspruch 1 angegebenen DNA-Sequenz hybridisiert und ein Protein mit der Immunreaktivität von HBeAg kodiert.

6. Rekombinanter Hefe-Expressionsvektor nach Anspruch 1, bei dem die in Hefe replizierbare DNA-Sequenz die 2µ- oder ars-DNA-Sequenz ist.

7. Rekombinanter Hefe-Expressionsvektor nach Anspruch 1, bei dem der Hefe-Promotor der ADH-2, GAL-, oder CYC1-Promotor oder ein daraus zusammengesetzter Hybrid-Promotor ist .

8. Rekombinanter Hefe-Expressionsvektor nach Anspruch 1, bei dem die DNA-Sequenz (e) eine Aminosäuredefizienz komplementiert und vorzugsweise das LEU2-oder URA3-Gen ist.

9. Hefe, die einen rekombinanten Hefe-Expressionsvektor nach Anspruch 1 enthält.

10. Hefe nach Anspruch 9, die zur Gattung Schizosaccharomyces oder Saccharomyces, vorzugsweise zur Art Schizosaccharomyces pombe oder Saccharomyces cerevisiae gehört.

11. Verfahren zur Herstellung eines Proteins mit der Immunreaktivität des HBeAg, bei dem man eine Hefe nach Anspruch 9 unter geeigneten Bedingungen züchtet und das Protein mit der Immunreaktivität des HBeAg aus der Kultur isoliert.

12. Diagnostisches Reagenz, das ein Protein mit der Immunreaktivität des HBeAg hergestellt nach Anspruch 11 enthält, das gegebenenfalls mit einer nachweisbaren Markierung, vorzugsweise einer radioaktiven, enzymatischen, fluoreszierenden oder chemolumineszenten Markierung, verknüpft ist.

13. Diagnostisches Reagenz nach Anspruch 12 zur Verwendung bei der Durchführung von Immunoassays.

14. Arzneimittel oder Impfstoff, enthaltend ein Protein mit der Immunreaktivität des HBeAg, hergestellt nach Anspruch 11, gegebenenfalls in Kombination mit einem pharmazeutisch verträglichen Träger und/oder Verdünnungsmittel und/oder weiteren Immunogenen.

15. Verwendung eines Proteins mit der Immunreaktivität des HBeAg, hergestellt nach Anspruch 11, zur Herstellung von polyklonalen oder monoklonalen Antikörpern.

## Patentansprüche (Patentansprüche für folgende(n) Vertragsstaat(en): ES)

1. Verfahren zur Herstellung eines rekombinanten Hefe-Expressionsvektors, der die folgenden Merkmale aufweist:
(a) eine in Hefe replizierbare DNA-Sequenz;
(b) einen Hefe-Promotor;
(c) eine DNA-Sequenz, die ein Protein mit der Immunreaktivität des HBeAg kodiert;
(d) ein Transkriptions-Stop-Signal; und
(e) eine DNA-Sequenz, die einen Hefe-Selektionsmarker kodiert,
dadurch gekennzeichnet, daß die DNA-Sequenz (c) die Aminosäuresequenz von der Position -29 der pre C-Sequenz bis zur Position +149 der C-Sequenz kodiert.

2. Verfahren nach Anspruch 1, dadurch gekennzeichnet, daß ein
(f) einen Replikationsursprung für Bakterien; und
(g) eine DNA-Sequenz, die einen bakteriellen Selektionsmarker kodiert, verwendet werden.

3. Verfahren nach Anspruch 1, dadurch gekennzeichnet, daß besagter Hefe-Expressionsvektor zusätzlich das Signalpeptid des Killertoxins von Kluyveromyces lactis oder einen biologisch aktiven Teil dieses Signalpeptids enthält.

4. Verfahren nach nach Anspruch 3, wobei das Signalpeptid des Killertoxins von Kluyveromyces lactis die folgende Aminosäure-Sequenz aufweist:

Met-Asn-Ile-Phe-Tyr-Ile-Phe-Leu-Phe-Leu-Leu-Ser-Phe-Val-Gln-Gly

5. Verfahren nach Anspruch 1, wobei die DNA-Sequenz (c) eine DNA-Sequenz ist, die mit einer in Anspruch 1 angegebenen DNA-Sequenz hybridisiert und ein Protein mit der Immunreaktivität von HBeAg kodiert.

6. Verfahren nach Anspruch 1, bei dem die in Hefe replizierbare DNA-Sequenz die 2µ- oder ars-DNA-Sequenz ist.

7. Verfahren nach Anspruch 1, bei dem der Hefe-Promotor der ADH-2, GAL-, oder CYC1-Promotor oder ein daraus zusammengesetzter Hybrid-Promotor ist.

8. Verfahren nach Anspruch 1, bei dem die DNA-Sequenz (e) eine Aminosäuredefizienz komplementiert und vorzugsweise das LEU2- oder URA3-Gen ist.

9. Verfahren zur Herstellung einer transformierten Hefe, die einen rekombinanten Hefe-Expressionsvektor nach Anspruch 1 enthält.

10. Verfahren nach Anspruch 9, dadurch gekennzeichnet, daß die Hefe zur Gattung Schizosaccharomyces oder Saccharomyces, vorzugsweise zur Art Schizosaccharomyces pombe oder Saccharomyces cerevisiae gehört .

11. Verfahren zur Herstellung eines Proteins mit der Immunreaktivität des HBeAg, bei dem man eine Hefe nach Anspruch 9 unter geeigneten Bedingungen züchtet und das Protein mit der Immunreaktivität des HBeAg aus der Kultur isoliert.

12. Verfahren zur Herstellung eines diagnostisches Reagenzes, dadurch gekennzeichnet, daß ein Protein mit der Immunreaktivität des HBeAg hergestellt nach Anspruch 11 enthält, das gegebenenfalls mit einer nachweisbaren Markierung, vorzugsweise einer radioaktiven, enzymatischen, fluoreszierenden oder chemolumineszenten Markierung, verknüpft ist.

13. Verfahren zur Herstellung eines diagnostisches Reagenzes nach Anspruch 12 zur Verwendung bei der Durchführung von Immunoassays.

14. Verfahren zur Herstellung eines Arzneimittels oder Impfstoffes, enthaltend ein Protein mit der Immunreaktivität des HBeAg, hergestellt nach Anspruch 11, gegebenenfalls in Kombination mit einem pharmazeutisch verträglichen Träger und/oder Verdünnungsmittel und/oder weiteren Immunogenen.

15. Verwendung eines Proteins mit der Immunreaktivität des HBeAg, hergestellt nach Anspruch 11, zur Herstellung von polyklonalen oder monoklonalen Antikörpern.

## Patentansprüche (Patentansprüche für folgende(n) Vertragsstaat(en): GR)

1. Rekombinanter Hefe-Expressionsvektor, der die folgenden Merkmale aufweist:
(a) eine in Hefe replizierbare DNA-Sequenz;
(b) einen Hefe-Promotor;
(c) eine DNA-Sequenz, die ein Protein mit der Immunreaktivität des HBeAg kodiert;
(d) ein Transkriptions-Stop-Signal; und
(e) eine DNA-Sequenz, die einen Hefe-Selektionsmarker kodiert,
dadurch gekennzeichnet, daß die DNA-Sequenz (c) die Aminosäuresequenz von der Position -29 der pre C-Sequenz bis zur Position +149 der C-Sequenz kodiert.

2. Rekombinanter Hefe-Expressionsvektor nach Anspruch 1, der außerdem die folgenden Merkmale aufweist:
(f) einen Replikationsursprung für Bakterien; und
(g) eine DNA-Sequenz, die einen bakteriellen Selektionsmarker kodiert.

3. Rekombinanter Hefe-Expressionsvektor nach Anspruch 1, dadurch gekennzeichnet, daß er zusätzlich das Signalpeptid des Killertoxins von Kluyveromyces lactis oder einen biologisch aktiven Teil dieses Signalpeptids enthält.

4. Rekombinanter Hefe-Expressionsvektor nach Anspruch 3, wobei das Signalpeptid des Killertoxins von Kluyveromyces lactis die folgende Aminosäure-Sequenz aufweist:

Met-Asn-Ile-Phe-Tyr-Ile-Phe-Leu-Phe-Leu-Leu-Ser-Phe-Val-Gln-Gly

5. Rekombinanter Hefe-Expressionsvektor nach Anspruch 1, wobei die DNASequenz (c) eine DNA-Sequenz ist, die mit einer in Anspruch 1 angegebenen DNA-Sequenz hybridisiert und ein Protein mit der Immunreaktivität von HBeAg kodiert.

6. Rekombinanter Hefe-Expressionsvektor nach Anspruch 1, bei dem die in Hefe replizierbare DNA-Sequenz die 2µ- oder ars-DNA-Sequenz ist.

7. Rekombinanter Hefe-Expressionsvektor nach Anspruch 1, bei dem der HefePromotor der ADH-2, GAL-, oder CYC1-Promotor oder ein daraus zusammengesetzter Hybrid-Promotor ist.

8. Rekombinanter Hefe-Expressionsvektor nach Anspruch 1, bei dem die DNASequenz (e) eine Aminosäuredefizienz komplementiert und vorzugsweise das LEU2- oder URA3-Gen ist.

9. Hefe, die einen rekombinanten Hefe-Expressionsvektor nach Anspruch 1 enthält.

10. Hefe nach Anspruch 9, die zur Gattung Schizosaccharomyces oder Saccharomyces, vorzugsweise zur Art Schizosaccharomyces pombe oder Saccharomyces cerevisiae gehört.

11. Verfahren zur Herstellung eines Proteins mit der Immunreaktivität des HBeAg, bei dem man eine Hefe nach Anspruch 9 unter geeigneten Bedingungen züchtet und das Protein mit der Immunreaktivität des HBeAg aus der Kultur isoliert.

12. Diagnostisches Reagenz, das ein Protein mit der Immunreaktivität des HBeAg hergestellt nach Anspruch 11 enthält, das gegebenenfalls mit einer nachweisbaren Markierung, vorzugsweise einer radioaktiven, enzymatischen, fluoreszierenden oder chemolumineszenten Markierung, verknüpft ist.

13. Diagnostisches Reagenz nach Anspruch 12 zur Verwendung bei der Durchführung von Immunoassays.

14. Verfahren zur Herstellung eines Arzneimittels oder Impfstoffes, enthaltend ein Protein mit der Immunreaktivität des HBeAg, hergestellt nach Anspruch 11, gegebenenfalls in Kombination mit einem pharmazeutisch verträglichen Träger und/oder Verdünnungsmittel und/oder weiteren Immunogenen.

15. Verwendung eines Proteins mit der Immunreaktivität des HBeAg, hergestellt nach Anspruch 11, zur Herstellung von polyklonalen oder monoklonalen Antikörpern.

## Claims (Claims for the following Contracting State(s): BE, CH, DE, DK, FR, GB, IT, LI, LU, NL, PT, SE)

1. A recombinant yeast expression vector which has the following features:
(a) a DNA sequence which is replicable in yeast;
(b) a yeast promoter;
(c) a DNA sequence which encodes a protein with the immunoreactivity of HBeAg;
(d) a transcription stop signal; and
(e) a DNA sequence which encodes a yeast selection marker,
wherein the DNA sequence (c) encodes the amino acid sequence from position -29 of the pre C sequence to position +149 of the C sequence.

2. A recombinant yeast expression vector as claimed in claim 1, which additionally has the following features:
(f) an origin of replication for bacteria; and
(g) a DNA sequence which encodes a bacterial selection marker.

3. A recombinant yeast expression vector as claimed in claim 1, which additionally contains the signal peptide of the killer toxin of Kluyveromyces lactis or a biologically active part of this signal peptide.

4. A recombinant yeast expression vector as claimed in claim 3, where the signal peptide of the killer toxin of Kluyveromyces lactis has the following amino-acid sequence:

Met-Asn-Ile-Phe-Tyr-Ile-Phe-Leu-Phe-Leu-Leu-Ser-Phe-Val-
Gln-Gly

5. A recombinant yeast expression vector as claimed in claim 1, where the DNA sequence (c) is a DNA sequence which hybridizes with a DNA sequence indicated in claim 1 and encodes a protein with the immunoreactivity of HBeAg.

6. A recombinant yeast expression vector as claimed in claim 1, where the DNA sequence which is replicable in yeast is the 2µ or ars DNA sequence.

7. A recombinant yeast expression vector as claimed in claim 1, where the yeast promoter is the ADH-2, GAL or CYC1 promoter or a hybrid promoter composed thereof.

8. A recombinant yeast expression vector as claimed in claim 1, where the DNA sequence (e) complements an amino-acid deficiency and is preferably the LEU2 or URA3 gene.

9. A yeast which contains a recombinant yeast expression vector as claimed in claim 1.

10. A yeast as claimed in claim 9, which belongs to the genus Schizosaccharomyces or Saccharomyces, preferably to the species Schizosaccharomyces pombe or Saccharomyces cerevisiae.

11. A process for the preparation of a protein with the immunoreactivity of HBeAg, in which a yeast as claimed in claim 9 is cultured under suitable conditions, and the protein with the immunoreactivity of HBeAg is isolated from the culture.

12. A diagnostic reagent which contains a protein with the immunoreactivity of HBeAg prepared as claimed in claim 11, which is linked where appropriate to a detectable label, preferably a radioactive, enzymatic, fluorescent or chemoluminescent label.

13. A diagnostic reagent as claimed in claim 12 for use when carrying out immunoassays.

14. A medicinal product or vaccine containing a protein with the immunoreactivity of HBeAg, prepared as claimed in claim 11, where appropriate in combination with a pharmaceutically acceptable carrier and/or diluent and/or further immunogens.

15. The use of a protein with the immunoreactivity of HBeAg, prepared as claimed in claim 11, for the preparation of polyclonal or monoclonal antibodies.

## Claims (Claims for the following Contracting State(s): ES)

1. A process for the preparation of a recombinant yeast expression vector which has the following features:
(a) a DNA sequence which is replicable in yeast;
(b) a yeast promoter;
(c) a DNA sequence which encodes a protein with the immunoreactivity of HBeAg;
(d) a transcription stop signal; and
(e) a DNA sequence which encodes a yeast selection marker,
wherein the DNA sequence (c) encodes the amino acid sequence from position -29 of the pre C sequence to position +149 of the C sequence.

2. A process as claimed in claim 1, wherein:
(f) an origin of replication for bacteria; and
(g) a DNA sequence which encodes a bacterial selection marker, are used.

3. A process as claimed in claim 1, wherein said yeast expression vector additionally contains the signal peptide of the killer toxin of Kluyveromyces lactis or a biologically active part of this signal peptide.

4. A process as claimed in claim 3, where the signal peptide of the killer toxin of Kluyveromyces lactis has the following amino-acid sequence:

Met-Asn-Ile-Phe-Tyr-Ile-Phe-Leu-Phe-Leu-Leu-Ser-Phe-Val-
Gln-Gly

5. A process as claimed in claim 1, where the DNA sequence (c) is a DNA sequence which hybridizes with a DNA sequence indicated in claim 1 and encodes a protein with the immunoreactivity of HBeAg.

6. A process as claimed in claim 1, where the DNA sequence which is replicable in yeast is the 2µ or ars DNA sequence.

7. A process as claimed in claim 1, where the yeast promoter is the ADH-2, GAL or CYC1 promoter or a hybrid promoter composed thereof.

8. A process as claimed in claims 1, where the DNA sequence (e) complements an amino-acid deficiency and is preferably the LEU2 or URA3 gene.

9. A process for the preparation of a transformed yeast which contains a recombinant yeast expression vector as claimed in claim 1.

10. A process as claimed in claim 9, wherein the yeast belongs to the genus Schizosaccharomyces or Saccharomyces, preferably to the species Schizosaccharomyces pombe or Saccharomyces cerevisiae.

11. A process for the preparation of a protein with the immunoreactivity of HBeAg, in which a yeast as claimed in claim 9 is cultured under suitable conditions, and the protein with the immunoreactivity of HBeAg is isolated from the culture.

12. A process for the preparation of a diagnostic reagent which contains a protein with the immunoreactivity of HBeAg prepared as claimed in claim 11, which is linked where appropriate to a detectable label, preferably a radioactive, enzymatic, fluorescent or chemoluminescent label.

13. A process for the preparation of a diagnostic reagent as claimed in claim 12 for use when carrying out immunoassays.

14. A process for the preparation of a medicinal product or vaccine containing a protein with the immunoreactivity of HBeAg, prepared as claimed in claim 14, where appropriate in combination with a pharmaceutically acceptable carrier and/or diluent and/or further immunogens.

15. The use of a protein with the immunoreactivity of HBeAg, prepared as claimed in claim 11, for the preparation of polyclonal or monoclonal antibodies.

## Claims (Claims for the following Contracting State(s): GR)

1. A recombinant yeast expression vector which has the following features:
(a) a DNA sequence which is replicable in yeast;
(b) a yeast promoter;
(c) a DNA sequence which encodes a protein with the immunoreactivity of HBeAg;
(d) a transcription stop signal; and
(e) a DNA sequence which encodes a yeast selection marker,
wherein the DNA sequence (c) encodes the amino acid sequence from position -29 of the pre C sequence to position +149 of the C sequence.

2. A recombinant yeast expression vector as claimed in claim 1, which additionally has the following features:
(f) an origin of replication for bacteria; and
(g) a DNA sequence which encodes a bacterial selection marker.

3. A recombinant yeast expression vector as claimed in claim 1, which additionally contains the signal peptide of the killer toxin of Kluyveromyces lactis or a biologically active part of this signal peptide.

4. A recombinant yeast expression vector as claimed in claim 3, where the signal peptide of the killer toxin of Kluyveromyces lactis has the following amino-acid sequence:

Met-Asn-Ile-Phe-Tyr-Ile-Phe-Leu-Phe-Leu-Leu-Ser-Phe-Val-
Gln-Gly

5. A recombinant yeast expression vector as claimed in claim 1, where the DNA sequence (c) is a DNA sequence which hybridizes with a DNA sequence indicated in claim 1 and encodes a protein with the immunoreactivity of HBeAg.

6. A recombinant yeast expression vector as claimed in claim 1, where the DNA sequence which is replicable in yeast is the 2µ or ars DNA sequence.

7. A recombinant yeast expression vector as claimed in claim 1, where the yeast promoter is the ADH-2, GAL or CYC1 promoter or a hybrid promoter composed thereof.

8. A recombinant yeast expression vector as claimed in claim 1, where the DNA sequence (e) complements an amino-acid deficiency and is preferably the LEU2 or URA3 gene.

9. A yeast which contains a recombinant yeast expression vector as claimed in claim 1.

10. A yeast as claimed in claim 9, which belongs to the genus Schizosaccharomyces or Saccharomyces, preferably to the species Schizosaccharomyces pombe or Saccharomyces cerevisiae.

11. A process for the preparation of a protein with the immunoreactivity of HBeAg, in which a yeast as claimed in claim 9 is cultured under suitable conditions, and the protein with the immunoreactivity of HBeAg is isolated from the culture.

12. A diagnostic reagent which contains a protein with the immunoreactivity of HBeAg prepared as claimed in claim 11, which is linked where appropriate to a detectable label, preferably a radioactive, enzymatic, fluorescent or chemoluminescent label.

13. A diagnostic reagent as claimed in claim 12 for use when carrying out immunoassays.

14. A process for the preparation of a medicinal product or vaccine containing a protein with the immunoreactivity of HBeAg, prepared as claimed in claim 11, where appropriate in combination with a pharmaceutically acceptable carrier and/or diluent and/or further immunogens.

15. The use of a protein with the immunoreactivity of HBeAg, prepared as claimed in claim 11, for the preparation of polyclonal or monoclonal antibodies.

## Revendications (Revendications pour l'(les) Etat(s) contractant(s) suivant(s): BE, CH, DE, DK, FR, GB, IT, LI, LU, NL, PT, SE)

1. Vecteur d'expression recombinant dans une levure, qui présente les caractères suivants
(a) une séquence d'ADN apte à la réplication dans une levure ;
(b) un promoteur de levure ;
(c) une séquence d'ADN qui code pour une protéine ayant la réactivité immunologique du HBeAg;
(d) un signal d'arrêt de transcription ; et
(e) une séquence d'ADN qui code un marqueur de sélection de levure,
caractérisé en ce que la séquence d'ADN (c) code la séquence d'aminoacides de la position -29 de la préséquence C à la position +149 de la séquence C.

2. Vecteur d'expression recombinant dans une levure selon la revendication 1, qui présente en outre les caractères suivante:
(f) une origine de réplication pour bactéries ; et
(g) une séquence d'ADN qui code pour un marqueur de sélection bactérien.

3. Vecteur d'expression recombinant dans une levure selon la revendication 1, caractérisé en ce qu'il contient en outre le peptide signal de la toxine tueuse de *Kluyveromyces lactis* ou une partie biologiquement active de ce peptide signal.

4. Vecteur d'expression recombinant dans une levure selon la revendication 3, dans lequel le peptide signal de la toxine tueuse de *Kluyveromyces lactis* présente la séquence d'aminoacides suivante :

Met-Asn-Ile-Phe-Tyr-Ile-Phe-Leu-Phe-Leu-Leu-Ser-Phe-Val-Gln-Gly

5. Vecteur d'expression recombinant dans une levure selon la revendication 1, dans lequel la séquence d'ADN (c) est une séquence d'ADN qui s'hybride avec une séquence d'ADN indiquée dans la revendication 1 et code pour une protéine ayant la réactivité immunologique de HBeAg.

6. Vecteur d'expression recombinant dans une levure selon la revendication 1, dans lequel la séquence d'ADN apte à la réplication dans une levure est la séquence d'ADN 2µ ou ars.

7. Vecteur d'expression recombinant dans une levure selon la revendication 1, dans lequel le promoteur de levure est le promoteur ADH-2, GAL ou CYC1 ou un promoteur hybride composé à partir de ceux-ci.

8. Vecteur d'expression recombinant dans une levure selon la revendication 1, dans lequel la séquence d'ADN (e) compense par complément une déficience d'aminoacides et est de préférence le gène LEU2 ou URA3.

9. Levure qui contient un vecteur d'expression recombinant dans une levure selon la revendication 1.

10. Levure selon la revendication 9, qui appartient au genre *Schizosaccharomyces* ou *Saccharomyces,* de préférence à l'espèce *Schizosaccharomyces pombe* ou *Saccharomyces cerevisiae*.

11. Procédé pour la production d'une protéine ayant la réactivité immunologique du HBeAg, dans lequel on cultive dans des conditions appropriées une levure selon la revendication 9 et on isole à partir de la culture la protéine ayant la réactivité immunologique du HBeAg.

12. Réactif de diagnostic, qui contient une protéine ayant la réactivité immunologique du HBeAg, produite selon la revendication 11, qui est éventuellement rattachée à un marqueur détectable, de préférence un marqueur radioactif, enzymatique, fluorescent ou chimiluminescent.

13. Réactif de diagnostic selon la revendication 12, pour utilisation dans l'exécution de dosages immunologiques.

14. Médicament ou vaccin, contenant une protéine ayant la réactivité immunologique du HBeAg, produite selon la revendication 11, éventuellement en association avec un véhicule et/ou diluant pharmaceutiquement acceptable et/ou d'autres agents immunogènes.

15. Utilisation d'une protéine ayant la réactivité immunologique du HBeAg, produite selon la revendication 11, pour la production d'anticorps monoclonaux ou polyclonaux.

## Revendications (Revendications pour l'(les) Etat(s) contractant(s) suivant(s): ES)

1. Procédé pour la production d'un vecteur d'expression recombinant dans une levure qui présente les caractères suivants:
(a) une séquence d'ADN apte à la réplication dans une levure ;
(b) un promoteur de levure ;
(c) une séquence d'ADN qui code pour une protéine ayant la réactivité immunologique du HBeAg;
(d) un signal d'arrêt de transcription ; et
(e) une séquence d'ADN qui code un marqueur de sélection de levure,
caractérisé en ce que la séquence d'ADN (c) code la séquence d'aminoacides de la position -29 de la préséquence C à la position +149 de la séquence C.

2. Procédé selon la revendication 1, caractérisé en ce que l'on utilise:
(f) une origine de réplication pour bactéries ; et
(g) une séquence d'ADN qui code pour un marqueur de sélection bactérien.

3. Procédé selon la revendication 1, caractérisé en ce que ledit vecteur d'expression dans une levure contient en outre le peptide signal de la toxine tueuse de *Kluyveromyces lactis* ou une partie biologiquement active de ce peptide signal.

4. Procédé selon la revendication 3, dans lequel le peptide signal de la toxine tueuse de *Kluyveromyces lactis* présente la séquence d'aminoacides suivante:

Met-Asn-Ile-Phe-Tyr-Ile-Phe-Leu-Phe-Leu-Leu-Ser-Phe-Val-Gln-Gly

5. Procédé selon la revendication 1, dans lequel la séquence d'ADN (c) est une séquence d'ADN qui s'hybride avec une séquence d'ADN indiquée dans la revendication 1 et code pour une protéine ayant la réactivité immunologique de HBeAg.

6. Procédé selon la revendication 1, dans lequel la séquence d'ADN apte à la réplication dans une levure est la séquence d'ADN 2µ ou ars.

7. Procédé selon la revendication 1, dans lequel le promoteur de levure est le promoteur ADH-2, GAL ou CYC1 ou un promoteur hybride composé à partir de ceux-ci.

8. Procédé selon la revendication 1, dans lequel la séquence d'ADN (e) compense par complément une déficience d'aminoacides et est de préférence le gène LEU2 ou URA3.

9. Procédé pour la production d'une levure transformée qui contient un vecteur d'expression recombinant dans une levure selon la revendication 1.

10. Procédé selon la revendication 9, caractérisé en ce que la levure appartient au genre *Schizosaccharomyces* ou *Saccharomyces,* de préférence à l'espèce *Schizosaccharomyces pombe* ou *Saccharomyces cerevisiae*.

11. Procédé pour la production d'une protéine ayant la réactivité immunologique du HBeAg, dans lequel on cultive dans des conditions appropriées une levure selon la revendication 9 et on isole à partir de la culture la protéine ayant la réactivité immunologique du HBeAg.

12. Procédé pour la préparation d'un réactif de diagnostic, caractérisé en ce qu'il contient une protéine ayant la réactivité immunologique du HBeAg, produite selon la revendication 11, qui est éventuellement rattachée à un marqueur détectable, de préférence un marqueur radioactif, enzymatique, fluorescent ou chimiluminescent.

13. Procédé pour la préparation d'un réactif de diagnostic selon la revendication 12, pour utilisation dans l'exécution de dosages immunologiques.

14. Procédé pour la fabrication d'un médicament ou d'un vaccin, contenant une protéine ayant la réactivité immunologique du HBeAg, produite selon la revendication 11, éventuellement en association avec un véhicule et/ou diluant pharmaceutiquement acceptable et/ou d'autres agents immunogènes.

15. Utilisation d'une protéine ayant la réactivité immunologique du HBeAg, produite selon la revendication 11, pour la production d'anticorps monoclonaux ou polyclonaux.

## Revendications (Revendications pour l'(les) Etat(s) contractant(s) suivant(s): GR)

1. Vecteur d'expression recombinant dans une levure, qui présente les caractères suivants:
(a) une séquence d'ADN apte à la réplication dans une levure ;
(b) un promoteur de levure ;
(c) une séquence d'ADN qui code pour une protéine ayant la réactivité immunologique du HBeAg;
(d) un signal d'arrêt de transcription ; et
(e) une séquence d'ADN qui code un marqueur de sélection de levure,
caractérisé en ce que la séquence d'ADN (c) code la séquence d'aminoacides de la position -29 de la préséquence C à la position +149 de la séquence C.

2. Vecteur d'expression recombinant dans une levure selon la revendication 1, qui présente en outre les caractères suivants:
(f) une origine de réplication pour bactéries ; et
(g) une séquence d'ADN qui code pour un marqueur de sélection bactérien.

3. Vecteur d'expression recombinant dans une levure selon la revendication 1, caractérisé en ce qu'il contient en outre le peptide signal de la toxine tueuse de *Kluyveromyces lactis* ou une partie biologiquement active de ce peptide signal.

4. Vecteur d'expression recombinant dans une levure selon la revendication 3, dans lequel le peptide signal de la toxine tueuse de *Kluyveromyces lactis* présente la séquence d'aminoacides suivante :

Met-Asn-Ile-Phe-Tyr-Ile-Phe-Leu-Phe-Leu-Leu-Ser-Phe-Val-Gln-Gly

5. Vecteur d'expression recombinant dans une levure selon la revendication 1, dans lequel la séquence d'ADN (c) est une séquence d'ADN qui s'hybride avec une séquence d'ADN indiquée dans la revendication 1 et code pour une protéine ayant la réactivité immunologique de HBeAg.

6. Vecteur d'expression recombinant dans une levure selon la revendication 1, dans lequel la séquence d'ADN apte à la réplication dans une levure est la séquence d'ADN 2µ ou ars.

7. Vecteur d'expression recombinant dans une levure selon la revendication 1, dans lequel le promoteur de levure est le promoteur ADH-2, GAL ou CYC1 ou un promoteur hybride composé à partir de ceux-ci.

8. Vecteur d'expression recombinant dans une levure selon la revendication 1, dans lequel la séquence d'ADN (e) compense par complément une déficience d'aminoacides et est de préférence le gène LEU2 ou URA3.

9. Levure, qui contient un vecteur d'expression recombinant dans une levure selon la revendication 1.

10. Levure selon la revendication 9, qui appartient au genre *Schizosaccharomyces* ou *Saccharomyces,* de préférence à l'espèce *Schizosaccharomyces pombe* ou *Saccharomyces cerevisiae*.

11. Procédé pour la production d'une protéine ayant la réactivité immunologique du HBeAg, dans lequel on cultive dans des conditions appropriées une levure selon la revendication 9 et on isole à partir de la culture la protéine ayant la réactivité immunologique du HBeAg.

12. Réactif de diagnostic, qui contient une protéine ayant la réactivité immunologique du HBeAg, produite selon la revendication 11, qui est éventuellement rattachée à un marqueur détectable, de préférence un marqueur radioactif, enzymatique, fluorescent ou chimiluminescent.

13. Réactif de diagnostic selon la revendication 12, pour utilisation dans l'exécution de dosages immunologiques.

14. Procédé pour la fabrication d'un médicament ou d'un vaccin, contenant une protéine ayant la réactivité immunologique du HBeAg, produite selon la revendication il, éventuellement en association avec un véhicule et/ou diluant pharmaceutiquement acceptable et/ou d'autres agents immunogènes.

15. Utilisation d'une protéine ayant la réactivité immunologique du HBeAg, produite selon la revendication 11, pour la production d'anticorps monoclonaux ou polyclonaux.
